(19) Europäisches Patentamt
European Patent Office
Office européen des brevets

(11) Publication number: **0 530 815 A1**

# (12) EUROPEAN PATENT APPLICATION

(21) Application number: **92115108.0**

(51) Int. Cl.$^5$: **G03F 7/021**

(22) Date of filing: **03.09.92**

(30) Priority: **04.09.91 JP 224424/91**

(43) Date of publication of application:
**10.03.93 Bulletin 93/10**

(84) Designated Contracting States:
**DE GB NL**

(71) Applicant: **Fuji Photo Film Co., Ltd.**
**210 Nakanuma Minamiashigara-shi**
**Kanagawa-ken(JP)**

(72) Inventor: **Koike, Akinobu, c/o Fuji Photo Film Co., Ltd.**
**4000, Kawashiri, Yoshida-cho**
**Haibara-gun, Shizuoka-ken(JP)**
Inventor: **Kawauchi, Ikuo, c/o Fuji Photo Film Co., Ltd.**
**4000, Kawashiri, Yoshida-cho**
**Haibara-gun, Shizuoka-ken(JP)**

(74) Representative: **Patentanwälte Grünecker, Kinkeldey, Stockmair & Partner**
**Maximilianstrasse 58**
**W-8000 München 22 (DE)**

## (54) Presensitized plate for use in making lithographic printing plate.

(57) A presensitized plate for use in making a lithographic printing plate comprises a substrate provided thereon with a light-sensitive layer formed from a light-sensitive composition comprising a diazonium polymeric compound having two kinds of specific repeating units and a lipophilic polymeric compound having an acid value of not less than 20 and less than 250. The presensitized plate can provide a lithographic printing plate having excellent printing durability since the diazonium compound used has a high molecular weight. Moreover, since the diazonium compound has alkali-soluble groups and the lipophilic compound having an acid value of not less than 20 and less than 250 and easily soluble in an alkaline water is used as a binder, the presensitized plate has good developability and can provide a lithographic printing plate free of background contamination.

EP 0 530 815 A1

## BACKGROUND OF THE INVENTION

The present invention relates to a presensitized plate for use in making a lithographic printing plate (hereunder referred to as "PS plate").

A PS plate in general comprises a substrate such as an aluminum plate provided thereon with a layer of a light-sensitive composition and a lithographic printing plate can be obtained by irradiating the PS plate with actinic light rays such as UV rays through, for instance, a negative to polymerize or crosslink the portion irradiated with the light rays and to thus make the portion insoluble in a developer and then developing the PS plate with the developer to dissolve the non-irradiated portion thereof. In the resulting lithographic printing plate, the irradiated portion serves as an image area which repels water and receives an oily ink while the non-irradiated portion serves as a non-image area which receives water and repels an oily ink.

There have widely been used, as such light-sensitive composition, those comprising diazo resins such as a condensate of p-diazodiphenylamine with formaldehyde.

On the other hand, aqueous alkaline developer compositions used for developing these PS plates which make use of these diazo resins are, for instance, a developer composition which is an aqueous solution comprising benzyl alcohol, an anionic surfactant and a water-soluble sulfite as disclosed in J.P. KOKAI No. Sho 53-44202( = U.S.Patent No. 4,186,006).

All of these developer compositions comprise organic substances such as organic solvents and surfactants. However, the use of organic solvents generally suffers from various drawbacks. For instance, they are generally toxic, give out bad smells and become a cause of a fire. In addition, the waste thereof is restricted by the BOD requirement. Thus, the cost of the resulting lithogaphic printing plate increases.

An attempt for developing a PS plate which makes use of a diazo resin with a developer composition substantially free of these organic solvents is disclosed in U.S.Patent No. 4,467,027 or Canadian Patent No. 1,172,492.

The developers disclosed in the foregoing patents have been used for the development of positive-working PS plates comprising o-naphthoquinonediazide compounds as light-sensitive components and are strong alkaline water having a pH of not less than 12. However, if a PS plate comprising a diazo resin conventionally used and synthesized by condensation-polymerization is developed with a strongly alkaline developer, the image portion thereof is peeled off from the substrate, the resulting lithographic printing plate has insufficient printing durability and as a result, provides only poor numbers of copies.

Further J.P. KOKAI No. Hei 2-29650 discloses diazo resins to which affinity to an alkaline developer is imparted through co-condensation of p-diazodiphenylamine with a compound carrying a carboxyl group (such as p-hydroxybenzoic acid). However, such a diazo resin has a low molecular weight and the resulting lithographic printing plate always has insufficient printing durability.

In addition to the diazo resins obtained from p-diazodiphenylamine, U.S.Patent No. 4,215,041 discloses diazo compounds having polyester groups in the main chains, but the preparation thereof requires a quite special method and such resins cannot be commonly used. Moreover, the resulting light-sensitive composition causes reduction of sensitivity during storing the composition or during storing it after applying to a substrate because of easy cleavage of the ester group in the main chain and thus the value added thereof is substantially impaired.

To eliminate these drawbacks, U.S.Patent No. 4,581,313 proposes diazonium compounds having repeating units represented by the general formula (I) as will be defined below, but an imagewise exposed light-sensitive composition comprising the diazonium compound is not sufficiently developed with an alkaline developer, particularly in the development under dilute or weak alkaline conditions and the diazonium compound causes the formation of a film thereof on the surface of the substrate (so-called formation of diazo residue) in non-image areas during development, which leads to reduction of printing properties of the resulting printing plate.

## SUMMARY OF THE INVENTION

Accordingly, a principal object of the present invention is to provide a novel PS plate having a high sensitivity, good developability with respect to an alkaline developer and can provide a lithographic printing plate having excellent printing durability and free of the formation of diazo residue.

The inventors of this invention have conducted various studies, have found out that the foregoing object can effectively be accomplished by using a diazonium compound having two kinds of specific repeating units and a specific binder in combination and thus have completed the present invention.

According to the present invention, there is provided a PS plate which comprises a substrate provided thereon with a light-sensitive layer comprising a diazonium compound which is a copolymer having repeating units represented by the following general formulas (I) and (II) and a lipophilic polymeric compound having an acid value of not less than 20 and less than 250:

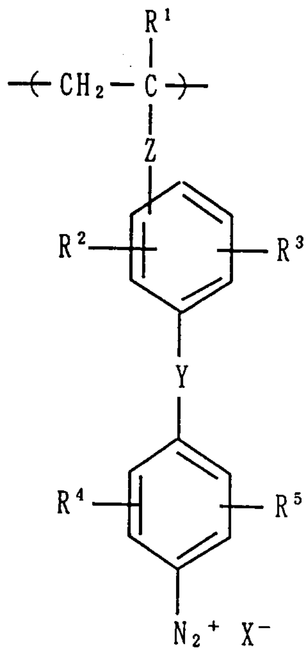

(I)

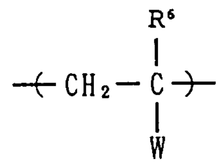

(II)

wherein $R^1$ and $R^6$ each represents a hydrogen atom or a methyl group; Z represents a divalent linking group; $R^2$, $R^3$, $R^4$ and $R^5$ may be the same or different and each represents a hydrogen or halogen atom or an alkyl, alkoxy or hydroxyl group; Y represents -NH-, -O- or -S-; X - represents an anion; W represents a carboxyl group, a group having at least one carboxyl group, a group having at least one sulfonate residue or a group having at least one phosphorus atom-containing oxyacid residue.

DESCRIPTION OF THE PREFERRED EMBODIMENTS

The present invention will hereinafter be explained in more detail.

In the foregoing formula (II), preferred phosphorus atom-containing oxyacid residues denoted by W are, for instance, those represented by the following general formulas (III) and (IV):

$-O-PO(OR^7)(OR^8)$ (III)

$-PO(OR^7)(OR^8)$ (IV)

wherein $R^7$ is a hydrogen atom; and $R^8$ represents a hydrogen atom or an alkyl or aryl group.

In the foregoing repeating units represented by Formula (I), Z is a divalent linking group and preferably a divalent linking group which forms the polymerizable moiety having an acrylic acid, methacrylic acid or styrene skeleton.

$R^2$, $R^3$, $R^4$ and $R^5$ may be the same or different and each preferably represents a hydrogen, chlorine or bromine atom, an alkyl or alkoxy group having 1 to 8 carbon atoms or a hydroxyl group.

Y is a divalent linking group such as -O-, -NH- or -S-with -NH- being preferred. $X^-$ is an anion which can form a stable salt with the diazonium compound used in the invention and makes the diazonium compound soluble in organic solvents. Such anions are, for instance, those derived from organic carboxylic acids such as decanoic acid and benzoic acid, organophosphoric acids such as phenylphosphoric acid and sulfonic acids and typical examples thereof include those derived from hydrohalogenic acids such as hydrofluoric acid, hydrochloric acid and hydrobromic acid; sulfuric acid, nitric acid, phosphoric acid (containing pentavalent phosphorus atom), in particular orthophosphoric acid; inorganic iso- and heteropoly

acids such as phosphotungstic acid and phosphomolybdic acid; aliphatic or aromatic phosphonic acids or half-esters thereof; arsonic acid, phosphinic acid, fluorocarboxylic acids such as trifluoroacetic acid; amidosulfonic acid, selenic acid, borofluoric acid, hexa-fluorophosphoric acid, perchloric acid; aliphatic and aromatic sulfonic acids such as methanesulfonic acid, fluoroalkanesulfonic acid, e.g., trifluoromethanesulfonic acid, laurylsulfonic acid, dioctylsulfosuccinic acid, dicyclohexylsulfosuccinic acid, camphorsulfonic acid, tolyloxy-3-propanesulfonic acid, nonylphenoxy-3-propanesulfonic acid, nonylphenoxy-4-butanesulfonic acid, dibutylphenoxy-3-propanesulfonic acid, diamylphenoxy-3-propanesulfonic acid, dinonylphenoxy-3-propanesulfonic acid, dibutylphenoxy-4-butanesulfonic acid, dinonylphenoxy-4-butanesulfonic acid, benzenesulfonic acid, toluenesulfonic acid, mesitylenesulfonic acid, p-chlorobenzenesulfonic acid, 2,5-dichlorobenzenesulfonic acid, sulfosalicylic acid, 2,5-dimethylbenzenesulfonic acid, p-acetylbenzenesulfonic acid, 5-nitro-o-toluenesulfonic acid, 2-nitrobenzenesulfonic acid, 3-chlorobenzenesulfonic acid, 3-bromobenzenesulfonic acid, 2-chloro-5-nitrobenzenesulfonic acid, butylbenzenesulfonic acid, octylbenzenesulfonic acid, dodecylbenzenesulfonic acid, butoxybenzenesulfonic acid, dodecyloxybenzenesulfonic acid, 2-methoxy-4-hydroxy-5-benzoylbenzenesulfonic acid, isopropylnaphthalenesulfonic acid, butylnaphthalenesulfonic acid, hexylnaphthalenesulfonic acid, octylnaphthalenesulfonic acid, butoxynaphthalenesulfonic acid, dodecyloxynaphthalenesulfonic acid, dibutylnaphthalenesulfonic acid, dioctylnaphthalenesulfonic acid, triisopropylnaphthalenesulfonic acid, tributylnaphthalenesulfonic acid, 1-naphthol-5-sulfonic acid, naphthalene-1-sulfonic acid, naphthalene-2-sulfonic acid, 1,8-dinitro-naphthalene-3,6-disulfonic acid, 4,4'-diazidostilbene-3,3'-disulfonic acid, 1,2-naphthoquinone-2-diazido-4-sulfonic acid, 1,2-naphthoquinone-2-diazido-5-sulfonic acid and 1,2-naphthoquinone-1-diazido-4-sulfonic acid. $X^-$ may be a mixture of the foregoing anions. Among these anions, particularly preferred are those derived from hexafluorophosphoric acid, dibutylnaphthalenesulfonic acid, dodecylbenzenesulfonic acid and 2-methoxy-4-hydroxy-5-benzoylbenzenesulfonic acid.

On the other hand, specific examples of the repeating units of Formula (II) include those derived from (meth)acrylic acid, vinylbenzoic acid, sulfopropyl methacrylate, sulfoethyl acrylate, sulfopropyl methacrylamide, sulfomethyl acrylamide, styrenesulfonic acid, 4-vinylphenylphosphate and 4-vinylphenylphosphonic acid, but the repeating units are not restricted to these examples.

The diazonium compounds used in the invention may comprise other copolymerizable monomer moieties other than the repeating units of Formulas (I) and (II).

Examples of copolymerizable monomers include alkyl ester of (meth)acrylic acid such as methyl methacrylate, ethyl acrylate, hydroxyethyl acrylate, propyl acrylate, cyclohexyl acrylate, 2-ethylhexyl acrylate, decyl acrylate, β -cyanoethyl acrylate, β -chloroethyl acrylate and 2-ethoxyethyl acrylate; vinyl esters such as vinyl acetate, vinyl propionate and vinyl butyrate; vinyl ethers such as methyl vinyl ether, butyl vinyl ether and oleyl vinyl ether; vinyl ketones such as methyl vinyl ketone and ethyl vinyl ketone; styrenes such as styrene, methylstyrene, dimethylstyrene, 2,4,6-trimethylstyrene, ethylstyrene, laurylstyrene, chlorostyrene, methoxystyrene, cyanostyrene, chloromethylstyrene andα -methylstyrene; vinyl heterocyclic compounds such as vinyl pyridine, vinyl pyrrolidone and vinyl imidazole; acrylonitrile, vinyl chloride, vinylidene chloride, ethylene, propylene, butadiene, diisobutylene, isoprene and chloroprene. The present invention is not restricted to the foregoing specific copolymerizable monomers and any monomer may be used so far as they are copolymerizable.

The copolymerization ratio and molecular weight may be arbitrarily be selected so far as the desired sensitivity, resolving power and solubility in organic solvents or the like of the diazonium compound are not impaired.

The copolymerization ratio of the repeating units (I) to the repeating units (II) ranges from 1:0.1 to 0.1:1, preferably 1:0.2 to 0.2:1 and more preferably 1:0.5 to 0.2:1 expressed in terms of charge ratio. Moreover, the acid content of the resulting diazonium compound (expressed in terms of the amount (meq.) of the alkali-soluble groups such as carboxylic acid, sulfonic acid and/or phosphorus atom-containing oxyacid residues included in 1g of the compound) ranges from 0.2 to 10 meq/g, preferably 0.5 to 5 meq/g and more preferably 0.5 to 3 meq/g.

The molecular weight of the compound may arbitrarily be controlled by variously changing the polymerization conditions, but the diazonium compound suitably has a weight-average molecular weight ranging from about 1,000 to 100,000 and preferably about 1,500 to 50,000.

Then specific examples of the repeating units represented by Formula (I) will be listed below, but the present invention is not limited to these specific ones.

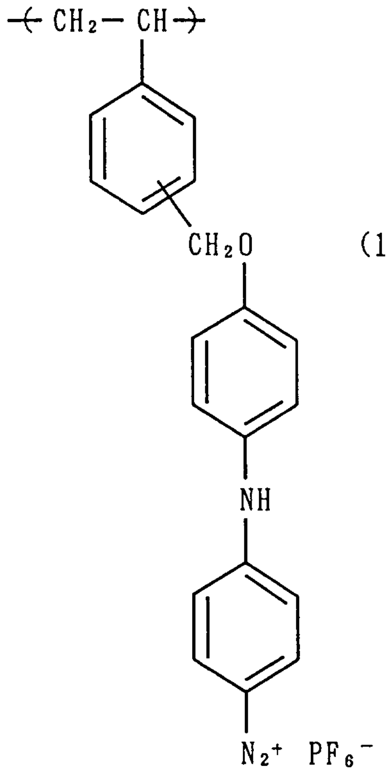

(1)

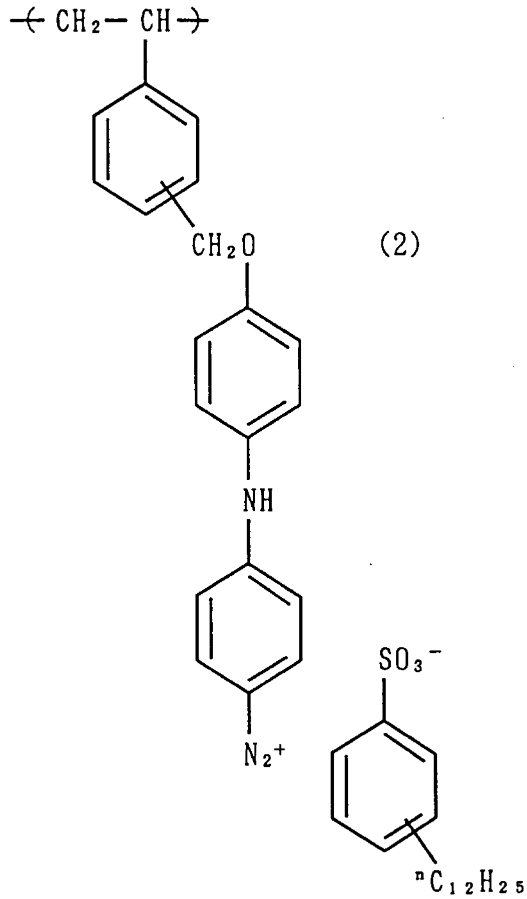

(2)

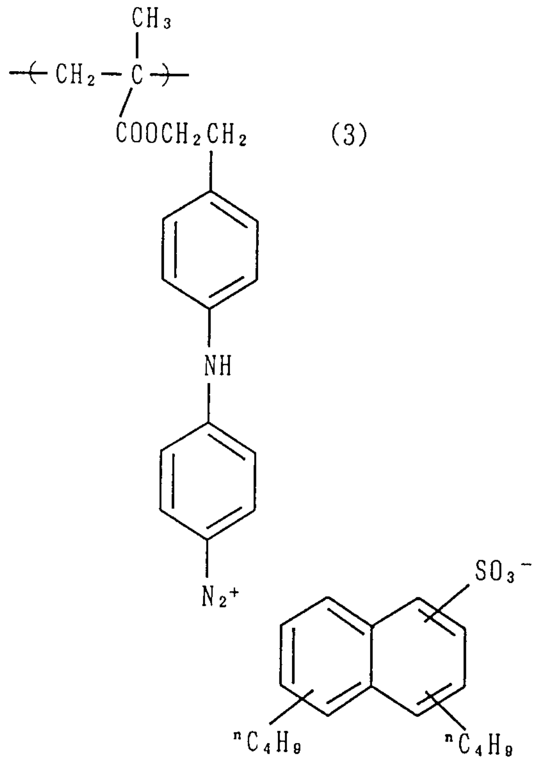

(3)

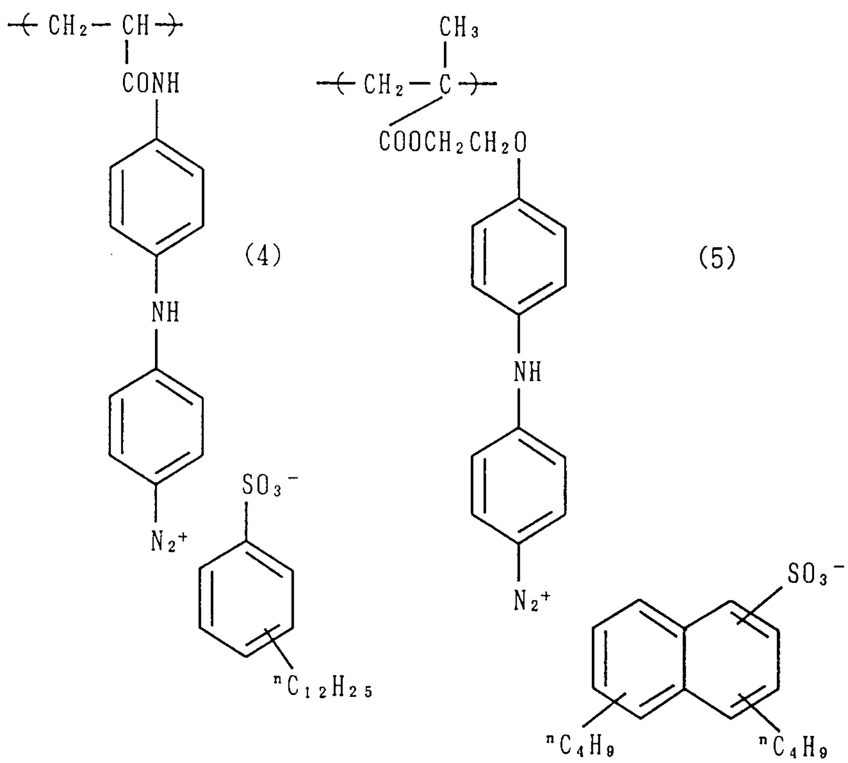

(4)

(5)

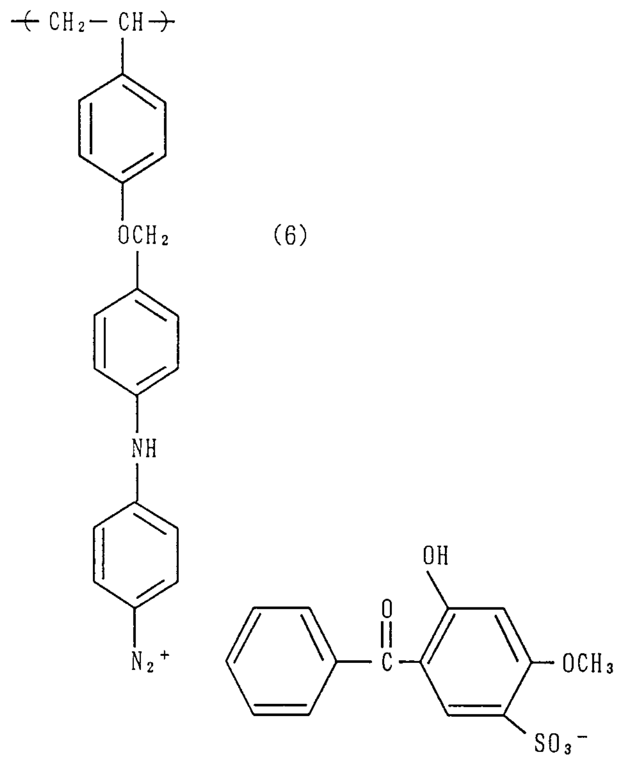

(6)

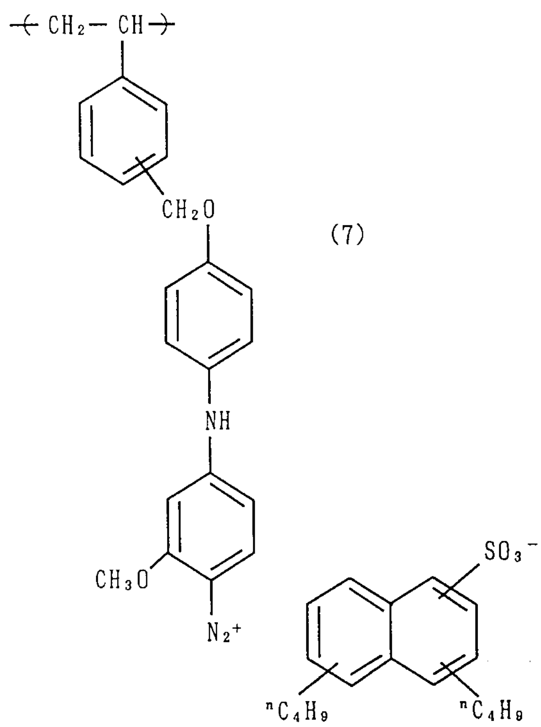

(7)

The repeating units represented by Formula (I) can be prepared by the following procedures. For instance, a compound having an active halogen atom and a polymerizable group such as a chloromethylstyrene derivative (a mixture of m- and p-isomers) represented by the following formula (V) is reacted with a diphenylamine derivative represented by the following general formula (VI) (wherein $R^2$, $R^3$, $R^4$, $R^5$ and Y are the same as those defined above in connection with Formula (I); and D represents a substituent capable

of deriving diazonium group such as $-NO_2$, -NO, $-N_2{}^+X^-$, $-NHCOCH_3$ or -H, or a group capable of being converted into a diazonium group through reduction or diazotization) according to, for instance, the method disclosed in Organic Functional Group Preparations, S.R. Sandler, W. Karo, Academic Press, Vol. 1, p. 102 to give a polymerizable monomer represented by the following general formula (VII).

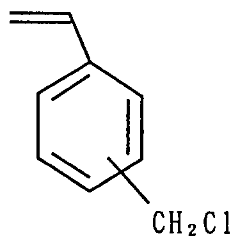

(V)

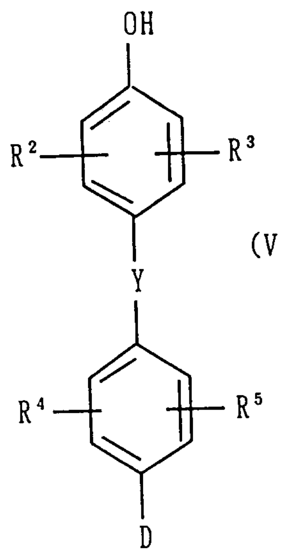

(VI)

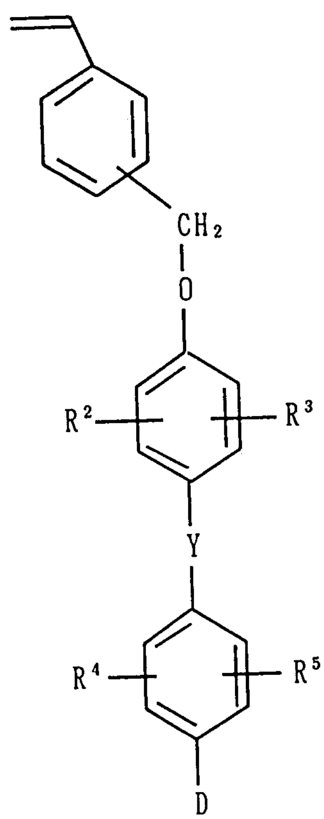

(VII)

Then the polymerizable monomer is polymerized in the presence of, for instance, an initiator for radical-polymerization or cationic polymerization in a solvent such as ethylene dichloride, cyclohexanone, methyl ethyl ketone, acetone, methanol, ethanol, ethylene glycol monomethyl ether, ethylene glycol monoethyl ether, 2-methoxyethyl acetate, 1-methoxy-2-propanol, 1-methoxy-2-propyl acetate, N,N-dimethylformamide, toluene, methyl acetate, methyl lactate or ethyl lactate to give a diazo polymer or a precursor thereof. The precursor can be converted into a diazo polymer in the usual manner. In particular, the diazotization of polymers having primary amino groups can be performed with reference to, for instance, Organic Functional Group Preparations, S.R. Sandler, W. Karo, Academic Press, Vol. 1, p. 404 and L.H. Kent et al., Biochem., 1960,77, p. 12.

The compound represented by Formula (VI) can be prepared by making use of, for instance, a method comprising condensing p-aminophenol with 2-chloro-5-nitrobenzenesulfonic acid and then subjecting the condensate to desulfonation (see, for instance, F. Ullmann et al., Ber., 42, p. 102; and F.L. Buch et al., J. Med. Chem., 1967, 10, p. 102) or a method comprising reacting 4-acetylamino-4'-methoxydiphenylamine with boron tribromide or the like to form a compound carrying a phenolic hydroxyl group (see, for instance,

J.F. McOmie et al., Org. Synth. V, p. 412; and R.J. Molyneux et al., Chem. Commun., 1974, p. 318).

The synthesis can also be performed by reacting (polymerizing) a polymer or copolymer of Formula (V) with a compound of Formula (VI). Thus, the present invention is not restricted to a specific synthesis method. Typical methods for preparing the compounds will be described below.

Preparation Example 1: Synthesis of Starting Materials

To a three-necked flask equipped with a stirring machine, a cooling tube and a thermometer, there were added 24.2 g (0.1 mole) of 4-oxy-4'-acetylaminodiphenylamine, 15.2 g (0.1 mole) of chloromethylstyrene (a mixture of m- and p-isomers), 6.9 g (0.05 mole) of potassium carbonate, 100 ml of N,N-dimethylformamide and 2 g of sodium iodide and the mixture was heated to 70 °C for 6 hours with stirring. After the reaction, the liquid reaction system was poured into 1ℓ of water to separate out the product. The product was recrystallized from ethanol to give the following polymerizable monomer.

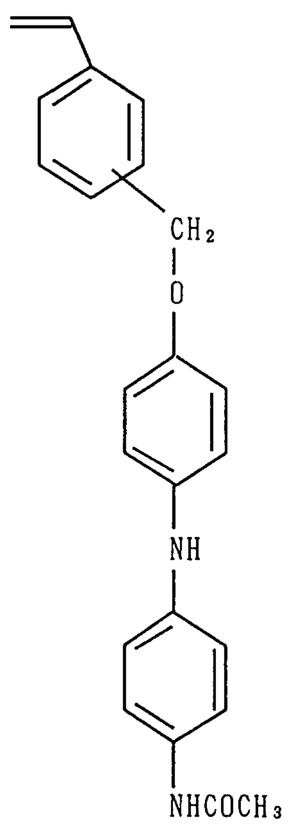

The yield of the monomer was 25.3 g (71%). The structure of the compound was confirmed by elemental analysis (N%: found 7.79; calculated 7.82), NMR spectroscopic measurement ($-COCH_3$ $\delta$ 2.0 ppm; $CH_2=CH-$ $\delta$ 5.23, 5.8 and 6.8 ppm; $-CH_2-O-$ $\delta$ 5.0 ppm; in $(CD_3)_2SO$) and IR spectroscopic measurement.

(Synthesis of Diazonium Compound 1)

To a three-necked flask equipped with a stirring machine, a cooling tube and a thermometer, there were added 17.9 g (0.0500 mole) of the polymerizable monomer prepared in Preparation Example 1, 4.30 g (0.0500 mole) of methacrylic acid and 35 g of N,N-dimethylformamide and the mixture was heated to 65 °C with stirring. After stirring the mixture for a while in a nitrogen gas atmosphere, a solution of 1.23 g of α, α'-azobisisobutyronitrile in 5 g of N,N-dimethylformamide was added and polymerization was carried out for 8 hours. Then the contents of the flask were poured into 2ℓ of water to separate out the product followed by filtration of the precipitated product and drying to give 21.2 g of a polymer.

The resulting polymer was introduced into a three-necked flask equipped with a stirring machine and a cooling tube, followed by addition of 40 ml of ethanol, 40 ml of water and 20 ml of conc. hydrochloric acid

and stirring at 75 °C for 6 hours. Thereafter, the contents of the flask were poured into 1 ℓ of acetone to give a powdery polymer. It was confirmed that the polymer had amino groups at p-positions and that the acetylamino groups were disappeared by the NMR and IR spectroscopic measurements.

Then the polymer, 35 ml of conc. hydrochloric acid and 160 ml of water were added to a three-necked flask provided with a stirring machine and a dropping funnel and the mixture was cooled to 0 °C . A solution of 3.45 g (0.0500 mole) of sodium nitrite in 20 ml of water was gradually dropwise added with vigorous stirring while maintaining the temperature of the reation system to not higher than 5 °C. Thereafter, stirring was further continued for additional one hour while maintaining the temperature of the reaction system to not higher than 5 °C. After completion of the reaction, insolubles formed were removed by filtration, the filtrate was dropwise added to a solution of 17.2 g of sodium dibutylnaphthalenesulfonate in 400 ml of water followed by removal of precipitated polymer through filtration and drying to give a diazo polymer (the diazonium compound (1) of the present invention).

The resulting diazonium compound was coupled with 1-phenyl-3-methyl-5-pyrazolone and then the molecular weight thereof was determined by the gel permeation chromatography (hereinafter referred to as "GPC") and was found to be 6200 on the average (weight-average).

(Preparation of Diazonium Compounds 2 to 13)

The same procedures used above were repeated to prepare the following diazonium compounds 2 to 13. In the preparation, the units (1) to (7) listed above were used as the repeating units represented by Formula (I) as shown in Table 1.

Table 1

| Diazo. | Rate of Monomers Present in Polymer | | | | | | | | | |
|---|---|---|---|---|---|---|---|---|---|---|
| Comp. | Repeating Unit (I) | | | | | | | Repeating Unit (II) | | MW$_{av}$ |
| No. | (1) | (2) | (3) | (4) | (5) | (6) | (7) | | | |
| 2 | 45 | | | | | | | methacrylic acid | 55 | 5420 |
| 3 | | 55 | | | | | | ditto | 45 | 6880 |
| 4 | | | 50 | | | | | ditto | 50 | 6110 |
| 5 | | | | 55 | | | | ditto | 45 | 7130 |
| 6 | | | | | 50 | | | ditto | 50 | 5920 |
| 7 | | | | | | 50 | | ditto | 50 | 5370 |
| 8 | | | | | | | 60 | ditto | 40 | 8140 |
| 9 | | 50 | | | | | | vinyl benzoate | 50 | 5650 |
| 10 | | 50 | | | | | | sulfopropyl methacrylate | 50 | 5710 |
| 11 | | 50 | | | | | | styrene sulfonic acid | 50 | 5890 |
| 12 | | 50 | | | | | | 4-vinylphenylphosphate | 50 | 6260 |
| 13 | | 50 | | | | | | 4-vinylphenylphosphonic acid | 50 | 6130 |

Then the following diazonium compounds 14 and 15 were prepared by way of comparison.

(Preparation of Diazonium Compound 14)

There was dissolved 14.5 g (50 mM) of p-diazodiphenylamine sulfate in 40.9 g of conc. sulfuric acid under ice-cooling. To the reaction solution, there was slowly dropwise added 1.5 g (50 mM) of paraformaldehyde while maintaining the temperature of the reaction system to not higher than 10 °C . Then stirring was continued for 2 hours under ice-cooling.

The reaction mixture was dropwise added to 50 ml of ethanol under ice-cooling and precipitates formed were filtered off. After washing the precipitates with ethanol, they were dissolved in 100 ml of pure water and a cold concentrated aqueous solution containing 6.8 g of zinc chloride was added to the solution. After filtration of the resulting precipitates, they were washed with ethanol and then dissolved in 150 ml of pure water. A cold concentrated aqueous solution of 16.8 g of sodium dibutylnaphthalenesulfonate was added to the solution. Precipitates formed were filtered off, followed by water-washing and drying at 30 °C over a day and night to give a diazonium compound 14.

The molecular weight of the diazonium compound 14 was determined by GPC and it was found that the compound comprised about 50 mole% of the pentamer.

(Preparation of Diazonium Compound 15)

There were dissolved, in 90 g of conc. sulfuric acid, 3.5 g (0.025 mole) of p-hydroxybenzoic acid and 22.0 g (0.025 mole) of 4-diazodiphenylamine sulfate under ice-cooling. To this solution, there was slowly added 2.7 g (0.09 mole) of paraformaldehyde while maintaining the temperature of the reaction system to not higher than 10°C . Thereafter, the reaction system was stirred for additional 2 hours under ice-cooling. The reaction mixture was poured into 1 ℓ of ethanol under ice-cooling and the resulting precipitates were filtered off. After washing with ethanol, the precipitates were dissolved in 200 ml of pure water and a cold concentrated solution of 10.5 g of zinc chloride in water was added to the solution. The resulting precipitates was filtered off, washed with ethanol and dissolved in 300 ml of pure water. A cold concentrated solution of 13.7 g of ammonium hexafluorophosphate in water was added to this solution. After filtration of the resulting precipitates and water-washing, they were dried at 30 °C over a day and night to give a diazonium compound 15.

The molecular weight of the diazonium compound 15 was determined by GPC and found to be about 1800 expressed in terms of weight-average molecular weight.

The light-sensitive composition used in the invention in general comprises 1 to 70% by weight, preferably 3 to 40% by weight of the diazonium compound on the basis of the weight of the solid contents of the composition.

The light-sensitive composition used in the invention may comprise, in addition to the diazonium compound of the invention, a diazonium compound of the conventional type prepared through polycondensation in an amount ranging from 0 to 50% by weight on the basis of the total weight of the diazonium compound.

Now referring to the lipophilic polymeric compound which is added to the light-sensitive layer of the light-sensitive material of the present invention in addition to the foregoing diazonium compound, the lipophilic polymeric compound can serve as a binder upon forming a light-sensitive composition. In the invention, the light-sensitive layer comprises a lipophilic polymeric compound having an acid value of not less than 20 and less than 250. This is because if the acid value thereof is less than 20, the resistance to contamination of the resulting printing plate is lowered while if it is 250 or higher, the resulting lithographic printing plate does not have sufficient printing durability.

The term "acid value" herein means the amount (mg) of potassium hydroxide required for neutralizing 1 g of the polymeric compound. The acid value can be determined as follows. A sample was diluted 50 times with methyl cellosolve and titrated with a 0.1 N potassium hydroxide aqueous solution. In this respect, the end point (point of neutralization) of the titration was set at the inflection point of the pH curve obtained by using a pH meter. The acid value can be calculated from the amount of the potassium hydroxide solution required for achieving the point of neutralization.

The lipophilic polymeric compounds usable in the invention are, for instance, copolymers having structural units derived from monomers selected from those listed below and having an acid value of not less than 20 and less than 250:

(1) (Meth)acrylamides, (meth)acrylic acid esters and hydroxystyrenes having an aromatic hydroxy group such as N-(4-hydroxyphenyl) (meth)acrylamide, o-, m- or p-hydroxystyrene and o-, m- or p-hydroxyphenyl-(meth)acrylate.

(2) Unsaturated sulfonamides such as m-aminosulfonylphenyl methacrylate, N-(p-aminosulfonylphenyl) (meth)acrylamide and N-(p-toluenesulfonyl) methacrylamide.

(3) (Meth)acrylic acid esters having an aliphatic hydroxy group such as 2-hydroxyethyl (meth)acrylate.

(4) Unsaturated carboxylic adids such as (meth)acrylic acid, maleic anhydride and itaconic acid.

(5) (Substituted) acrylates such as methyl acrylate, ethyl acrylate, propyl acrylate, butyl acrylate, amyl acrylate, hexyl acrylate, octyl acrylate, benzyl acrylate, phenyl acrylate, 2-chloroethyl acrylate, glycidyl acrylate and N-d imethylaminoethyl acrylate.

(6) (Substituted) methacrylates such as methyl methacrylate, ethyl methacrylate, propyl methacrylate, butyl methacrylate, amyl methacrylate, cyclohexyl methacrylate, benzyl methacrylate, phenyl methacrylate, 4-hydroxybutyl methacrylate, glycidyl methacrylate and N-dimethylaminoethyl methacrylate.

(7) (Meth)acrylamides such as (meth)acrylamide, N-methylol (meth)acrylamide, N-ethyl acrylamide, N-hexyl methacrylamide, N-cyclohexyl acrylamide, N-hydroxyethyl acrylamide, N-phenyl acrylamide, N-nitrophenyl acrylamide and N-ethyl-N-phenyl acrylamide.

(8) Vinyl ethers such as ethyl vinyl ether, 2-chloroethyl vinyl ether, hydroxyethyl vinyl ether, propyl vinyl ether, butyl vinyl ether, octyl vinyl ether and phenyl vinyl ether.

(9) Vinyl esters such as vinyl acetate, vinyl chloro acetate, vinyl butyrate and vinyl benzoate.

(10) Styrenes such as styrene, methylstyrene and chloromethylstyrene.

(11) Vinyl ketones such as methyl vinyl ketone, ethyl vinyl ketone, propyl vinyl ketone and phenyl vinyl ketone.

(12) Olefins such as ethylene, propylene, isobutylene, butadiene and isoprene.

(13) N-vinyl pyrrolidone, N-vinyl carbazole, 4-vinyl pyridine, acrylonitrile and methacrylonitrile.

(14) Unsaturated imides such as maleimide, N-acryloylacrylamide, N-acetylmethacrylamide, N-propionylmethacrylamide and N-(p-chlorobenzoyl)methacrylamide.

Further, the foregoing monomers may also be copolymerized with other monomers copolymerizable therewith and the copolymers of the monomers listed above can be modified with, for instance, glycidyl (meth)acrylate. However, the copolymers usable in the invention are not limited to these specific examples.

Examples of particularly preferred polymeric compounds are, for instance, copolymers comprising, as essential component, (meth)acrylic acid, crotonic acid or maleic acid such as multi-component copolymers of 2-hydroxyethyl (meth)acrylate/(meth) acrylonitrile/(meth)acrylic acid/optional copolymerizable other monomers as disclosed in U.S. Patent No. 4,123,276; multi-component copolymers of (meth)acrylic acid having terminal hydroxyl group and esterified with a group carrying dicarboxylic acid residue/(meth)acrylic acid/optional copolymerizable other monomers as disclosed in J.P. KOKAI No. Sho 53-120903; multi-component copolymers of monomers having aromatic hydroxyl group (such as N-(4-hydroxyphenyl)-methacrylamide)/(meth)acrylic acid/optional copolymerizable other monomers as disclosed in U.S. Patent No. 4,731,316; multi-component copolymers of alkyl acrylate/(meth)acrylonitrile/unsaturated carboxylic acids as disclosed in U.S. Patent No. 4,304,832; and multi-component copolymers having sulfonamido groups as disclosed in EP-330239 (A2).

The foregoing copolymers preferably comprises repeating units derived from the unsaturated carboxylic acids (4) defined above.

Moreover, preferred molecular weight of these copolymers ranges from 10,000 to 150,000. Examples of polymeric compounds preferably used in the invention further include polyurethanes which are made alkali-soluble as disclosed in Japanese Patent Publication for Opposition Purpose (hereinafter referred to as "J.P. KOKOKU") No. Sho 54-19773 ( = U.S.Patent No. 3,732,105) and U.S.Patent Nos. 4,387,151; 4,631,245; 4,741,985; 4,983,491; 4,877,711 and J.P.KOKAI Hei 2-146042.

Examples of solvents used in the preparation of these polymeric compounds are ethylene dichloride, cyclohexanone, methyl ethyl ketone, acetone, methanol, ethanol, ethylene glycol monomethyl ether, ethylene glycol monoethyl ether, 2-methoxyethyl acetate, 1-methoxy-2-propanol, 1-methoxy-2-propyl acetate, N,N-dimethylformamide, toluene, ethyl acetate, methyl lactate and ethyl lactate.

These lipophilic polymeric compounds may be used alone or in combination. These polymeric compounds can be incorporated into the light-sensitive composition in an amount generally ranging from 40 to 99% by weight, preferably 50 to 95% by weight on the basis of the weight of the solid contents of the composition.

The light-sensitive composition may optionally comprise resins such as polyvinyl butyral resins, polyamide resins, epoxy resins, novolak resins and/or natural resins.

Preparation Examples of the foregoing lipophilic polymeric compounds will be given below.

(Synthesis of Lipophilic Polymeric Compound 1)

To a 1ℓ three-necked flask equipped with a stirring machine, a cooling tube and a dropping funnel, there were added 170.2g (1.0 mole) of 4-aminobenzenesulfonamide and 700 ml of tetrahydrofuran and the mixture was stirred in an ice-bath. Methacrylic acid chloride (52.3 g; 0.5 mole) was dropwise added to the mixture through the dropping funnel over about one hour. After completion of the dropwise addition, the ice-bath was removed, followed by stirring at room temperature for 30 minutes and heating the mixture to 60°C in an oil bath with stirring over additional one hour. After the reaction, the reaction mixture was poured into 3ℓ of water with stirring, then stirred for 30 minutes and filtered to give N-(4-aminosulfonylphenyl)-methacrylamide as a white solid. The white solid could be purified by recrystallizing from a ethanol/acetone mixed solvent (yield 39.3g).

Then 8.47 g (0.0350 mole) of N-(4-aminosulfonylphenyl) methacrylamide, 2.55 g (0.0480 mole) of acrylonitrile, 3.01 g(0. 0350 mole) of methacrylic acid, 13.28 g (0.0820 mole) of benzyl acrylate, 0.41 g of α, α '-azobisisobutyronitrile and 25 g of N,N-dimethylformamide were introduced into a 100 ml three-necked flask equipped with a stirring machine and a cooling tube and the mixture was stirred for 5 hours under heating at 64 °C . This reaction mixture was poured into 2ℓ of water under stirring followed by stirring for 30 minutes, filtration and drying to give 22 g of a lipophilic polymeric compound 1.

This polymeric compound 1 was found to have an acid value of 143 and a weight-average molecular weight as determined by GPC of 32,000.

(Synthesis of Lipophilic Polymeric Compound 2)

To a 100 ml three-necked flask equipped with a stirring machine and a cooling tube, there were added 5.45 g (0.0240 mole) of N-(4-aminosulfonylphenyl)acrylamide, 6.25 g (0.0480 mole) of 2-hydroxyethyl methacrylate, 2.06 g (0.0240 mole) of methacrylic acid, 10.4 g (0.104 mole) of ethyl acrylate, 0.41 g of α, α '-azobisisobutyronitrile and 29 g of N,N-dimethylformamide and the mixture was stirred for 5 hours under heating at 64°C . This reaction mixture was poured into 2ℓ of water with stirring followed by stirring for 30 minutes, filtration and drying to give 19 g of a lipophilic polymeric compound 2.

This polymeric compound 2 was found to have an acid value of 111 and a weight-average molecular weight as determined by GPC of 27,000.

(Synthesis of Lipophilic Polymeric Compound 3)

There were dissolved, in 112 ml of a 1:1 acetone/methanol mixed solvent, 39 g of N-(4-hydroxyphenyl)-methacrylamide, 13 g of acrylonitrile, 51.8 g of benzyl acrylate, 18.9 g of methacrylic acid and 1.642 g of azobisisobutyronitrile, followed by replacement of the atmosphere with nitrogen and heating at 60 °C for 8 hours.

After the reaction, the reaction solution was poured into 5ℓ of water with stirring, the resulting white precipitates were filtered off and dried to give 99 g of a lipophilic polymeric compound 3.

This polymeric compound 3 was found to have an acid value of 175 and a weight-average molecular weight as determined by GPC of 65,000.

(Synthesis of Lipophilic Polymeric Compound 4)

To a 100 ml three-necked flask equipped with a stirring machine and a cooling tube, there were added 4.00 g (0.0754 mole) of acrylonitrile, 10.0 g (0.0768 mole) of 2-hydroxyethyl methacrylate, 1.00 g (0.0116 mole) of methacrylic acid, 5.00 g (0.0438 mole) of ethyl methacrylate, 0.41 g ofα, α '-azobisisobutyronitrile and 29 g of N,N-dimethylformamide and the mixture was stirred for 5 hours under heating at 64°C . This reaction mixture was poured into 2ℓ of water with stirring followed by stirring for 30 minutes, filtration and drying to give 19 g of a lipophilic polymeric compound 4.

This polymeric compound 4 was found to have an acid value of 32 and a weight-average molecular weight as determined by GPC of 33,000.

(Synthesis of Lipophilic Polymeric Compound 5)

To a 100 ml three-necked flask equipped with a stirring machine and a cooling tube, there were added 2.00 g (0.0113 mole) of N-(4-hydroxyphenyl)methacrylamide, 4.00 g (0.0307 mole) of 2-hydroxyethyl methacrylate, 2.00 g (0.0230 mole) of methacrylic acid, 7.00 g (0.069 mole) of methyl methacrylate, 5. 00 g

(0.0942 mole) of acrylonitrile, 0.41 g of α , α '-azobisisobutyronitrile and 29 g of N,N-dimethylformamide and the mixture was stirred for 5 hours under heating at 64° C . This reaction mixture was poured into 2ℓ of water with stirring followed by stirring for 30 minutes, filtration and drying to give 18 g of a lipophilic polymeric compound 5.

This polymeric compound 5 was found to have an acid value of 65 and a weight-average molecular weight as determined by GPC of 48,000.

The following lipophilic polymeric compounds were prepared by way of comparison.

(Synthesis of Lipophilic Polymeric Compound 6)

The same procedures used for the synthesis of the polymeric compound 4 were repeated except that 2.12 g (0.040 mole) of acrylonitrile, 13.00 (0.100 mole) of 2-hydroxyethyl methacrylate, 6.61 g (0.058 mole) of ethyl methacrylate and 0.17 g (0.002 mole) of methacrylic acid were used to give 17.5 g of a lipophilic polymeric compound 6. This polymeric compound 6 was found to have an acid value of 6.5 and a weight-average molecular weight as determined by GPC of 35,000.

(Synthesis of Lipophilic Polymeric Compound 7)

The same procedures used for the synthesis of the polymeric compound 1 were repeated except that 14.4 g (0.060 mole) of N-(4-aminosulfonylphenyl)methacrylamide, 1.86 g (0.035 mole) of acrylonitrile, 6.02 g (0.070 mole) of methacrylic acid and 5.67 g (0.035 mole) of benzyl acrylate were used to give 24. 2 g of a lipophilic polymeric compound 7. This polymeric compound 7 was found to have an acid value of 260 and a weight-average molecular weight as determined by GPC of 27,000.

The light-sensitive composition used in the invention may further comprise a dye for the purposes of obtaining a visible image (exposed visible image) after imagewise exposure to light and of obtaining a visible image after development.

Dyes preferably used in the invention are those causing a change in the color tone through reactions with free radicals or acids. The term "change in color tone" herein includes color tone changes from colorless to a color; and from colors to colorless or other colors. Preferred are dyes causing the color tone change through formation of salts with acids.

Specific examples of dyes which cause color tone changes from colors to colorless or other colors are triphenylmethane, diphenylmethane, oxazine, xanthene, iminonaphthoquinone, azomethine and anthraquinone type dyes represented by Victoria Pure Blue BOH (available from Hodogaya Chemical Co., Ltd.), Oil Blue #603 (available from Orient Chemical Industries, Ltd.), Patent Pure Blue (available from Sumitomo Mikuni Chemical Co., Ltd.), Crystal Violet, Brilliant Green, Ethyl Violet, Methyl Violet, Methyl Green, Erythrosine B, Basic Fuchsine, Malachite Green, Oil Red, m-Cresol Purple, Rhodamine B, Auramine, 4-p-diethylaminophenyliminaphthoqinone and cyano-p-diethylaminophenylacetamide.

On the other hand, examples of dyes which cause color tone changes from colorless to colors are leuco dyes and primary or secondary arylamine type dyes represented by triphenylamine, diphenylamine, o-chloroaniline, 1,2,3-triphenylguanidine, naphthylamine, diaminodiphenylmethane, p,p'-bis-dimethylaminodiphenylamine, 1,2-di-anilinoethylene, p,p',p''-tris-dimethylaminotriphenylmethane, p,p'-bis-dim ethylaminodiphenylmethylimine, p,p',p''-triamino-o-methyltriphenylmethane, p,p'-bis-dimethylaminodiphenyl-4-anilinonaphthylmethane, p,p',p''-triaminotriphenylmethane.

Particularly preferred are triphenylmethane and diphenylmethane type dyes and more preferably triphenylmethane type dyes, in particular, Victoria Pure Blue BOH.

These dyes are in general incorporated into the light-sensitive composition in an amount preferably ranging from about 0.5 to about 10% by weight and more preferably about 1 to 5% by weight on the basis of the total weight of the composition.

The light-sensitive composition used in the invention may further comprise other various additives. Preferred examples thereof include alkyl ethers for improving coating properties (e.g., ethyl cellulose and methyl cellulose), fluorine atom-containing surfactants and nonionic surfactants with the former being preferred, plasticizers for imparting flexibility and wear resistance to coated films (such as butyl phthalyl, polyethylene glycol, tributyl citrate, diethyl phthalate, dibutyl phthalate, dihexyl phthalate, dioctyl phthalate, tricresyl phosphate, tributyl phosphate, trioctyl phosphate, tetrahydrofurfuryl oleate, and oligomers and polymers of (meth) acrylic acid, with tricresyl phosphate being particularly preferred), sensitizing agents for improving the ink receptivity of image areas (for instance, half esters of styrene-maleic anhydride copolymer with alcohols as disclosed in U.S.Patent No. 4,294,905, novolak resins such as p-t-butylphenol/formaldehyde resin, and 50% fatty acid esters of p-hydroxystyrene), stabilizers (such as

phosphoric acid, phosphorous acid, and organic acids, for instance, citric acid, oxalic acid, dipicolinic acid, benzenesulfonic acid, naphthalenesulfonic acid, sulfosalicylic acid, 4-methoxy-2-hydroxybenzophenone-5-sulfonic acid and tartaric acid), and development accelerators (such as higher alcohols and acid anhydrides).

The amount of these additives varies depending on the kinds of the light-sensitive compositions and purposes, but in general ranges from 0.01 to 30% by weight on the basis of the weight of the solid contents of the composition.

The foregoing light-sensitive composition is dissolved in a proper solvent to give a coating solution of the composition, then applied onto a substrate and dried to give a PS plate. Examples of solvents usable in this process include methyl cellosolve, ethyl cellosolve, dimethoxyethane, diethylene glycol monomethyl ether, diethylene glycol dimethyl ether, 1-methoxy-2-propanol, methyl cellosolve acetate, acetone, methyl ethyl ketone, methanol, dimethylformamide, dimethylacetamide, cyclohexanone, dioxane, tetrahydrofuran, methyl lactate, ethyl lactate, ethylene dichloride, dimethylsulfoxide, water and mixture thereof.

These solvents may be used alone, but preferred are mixtures of high boiling point solvents such as methyl cellosolve, 1-methoxy-2-propanol and methyl lactate and low boiling point solvents such as methanol and methyl ethyl ketone.

The solid content of the coating solution preferably ranges from 1 to 50% by weight. In this case, the coated amount of the light-sensitive composition in general ranges from 0.2 to 10 $g/m^2$, preferably 0.5 to 3 $g/m^2$ (weighed after drying).

A substrate is used in the preparation of the PS plate of the present invention. Examples thereof include paper, paper laminated with a plastic film such as polyethylene, polypropylene or polystyrene film; a metal plate such as an aluminum (inclusive of aluminum alloys), zinc or copper plate; a plastic film such as a cellulose diacetate, cellulose triacetate, cellulose propionate, polyethylene terephthalate, polyethylene, polypropylene, polycarbonate or polyvinyl acetal film; paper or a plastic film listed above which is laminated with a metal foil or on which a layer of the foregoing metal is vapor-deposited; and copper plates plated with aluminum or chromium. Particularly preferred are an aluminum plate and composite substrate coated with aluminum.

The thickness of the aluminum plate preferably ranges from 0.1 to 0.6 mm. The PS plate of the presnt invention may be one-side type one or double-side type one whose both surfaces can be used through identical processings. Only the one-side type PS plate will be explained below for simplicity. In this respect, double-side type PS plates can be prepared by processing both surfaces thereof in the same manner.

The aluminum plate is desirably surface-treated for improving the water retention characteristics and adhesion to the light-sensitive layer subsequently applied thereto.

Examples of surface-roughening treatments are commonly known brush graining, ball graining, electrolytic etching, chemical etching, liquid honing, sandblasting and combination thereof, with the brush graining, electrolytic etching, chemical etching and liquid honing being preferred. Among these, particularly preferred are surface-roughening treatments comprising electrolytic etching. In addition, preferred also include methods in which an aluminum plate is subjected to brush-graining and then electrolytic etching as disclosed in U.S. Patent Nos. 4,476,006 and 4,477,317.

Examples of electrolytic baths are aqueous solutions containing acids, alkalis or salts thereof or those auqeous solutions which further comprise organic solvents. Among these, particularly preferred are electrolytes comprising hydrochloric acid, nitric acid or salts thereof.

The surface-roughened aluminum plate is optionally desmutted with an aqueous solution of an acid or alkali.

The aluminum plate thus obtained is desirably anodized preferably by a method comprising anodization in a bath containing sulfuric acid or phosphoric acid.

Further, the aluminum plate is optionally hydrophilized by subjecting it to a silicate (sodium silicate, potassium silicate) treatment as disclosed in U.S. Patent Nos. 2,714,066 and 3,181,461; a potassium fluorozirconate treatment as disclosed in U.S. Patent No. 2,946,638; a phosphomolybdate treatment as disclosed in U.S. Patent No. 3,201,247; an alkyl titanate treatment as disclosed in U.K. Patent No. 1, 108,559; a polyacrylic acid treatment as disclosed in German Patent No. 1, 091,433; a polyvinyl phosphonic acid treatment as disclosed in German Patent No. 1,134,093 and U.K. Patent No. 1,230,447; a phosphonic acid treatment as disclosed in J.P. KOKOKU No. Sho 44-6409; a phytic acid treatment as disclosed in U.S. PatentNo. 3,307,951; a treatment with a complex salt of a hydrophilic organic polymeric compound and a bivalent metal as disclosed in J.P. KOKAI Nos. Sho 58-16893 and Sho 58-18291.

Examples of other hydrophilization treatments are silicate electrodeposition treatments as disclosed in U.S. Patent No. 3,658,662.

It is also preferred to use an aluminum plate which is subjected to a sealing treatment after graining and anodization treatments. Such a sealing treatment can be performed by immersing the aluminum plate in hot water or a hot aqueous solution containing an inorganic or organic salt or by means of a steam bath.

The substrate preferably used in the present invention is obtained by immersing a 1S aluminum plate which comprises 0.1 to 0.5% of iron, 0.03 to 0.3% of silicon, 0.001 to 0.03% of copper and 0.002 to 0.1% of titanium in an alkaline aqueous solution, preferably 1 to 30% aqueous solutions of sodium hydroxide, potassium hydroxide, sodium carbonate and sodium silicate (the etching solution may comprise aluminum in an amount of 1/5 time that of the alkali) at a temperature of 20 to 80 °C for 5 to 250 seconds to perform etching and then dipping it in 10 to 30% aqueous solution of nitric acid or sulfuric acid at a temperature of 20 to 70 °C for 5 to 250 seconds to perform neutralization and removal of smuts after the alkali-etching.

After such a surface cleaning of the aluminum alloy plates, they are subsequently subjected to a surface-roughening treatment. Examples of surface-roughening treatments are brush graining or/and electrolytic etching treatments.

The brush graining treatment is preferably carried out using an aqueous suspension of pumice stone and a nylon brush and preferred surface-roughness ranges from 0.25 to 0.9$\mu$ .

Electrolyte solutions used in the electrolytic etching are aqueous solutions of hydrochloric acid or nitric acid and the concentration thereof preferably ranges from 0.01 to 3% by weight and more preferably 0.05 to 2.5% by weight.

The electrolytes may optionally contain corrosion inhibiting agents (or stabilizers) and/or agents for uniformizing grained surface such as nitrates, chlorides, monoamines, diamines, aldehydes, phosphoric acid, chromic acid, boric acid and ammonium oxalate. In addition, the electrolyte solution may comprise a proper amount (1 to 10 g/l) of aluminum ions.

The temperature of the electrolyte solution in general ranges from 10 to 60 °C during the treatment. The alternating current used in this treatment may be in any wave form such as rectangular wave, trapezoidal wave or sign wave so far as it comprises alternating positive and negative polarities and thus usual commercial single-phase and three-phase alternating current may be used. The current density in the electrolytic graining desirably ranges from 5 to 100 A/dm$^2$ and the treatment is desirably continued for 10 to 300 seconds.

The surface roughness of the aluminum alloy plates used in the present invention is controlled by adjusting the quantity of electricity so that it ranges from 0.2 to 0.8 $\mu$m.

The aluminum alloys thus surface grained are preferably treated with 10 to 50% hot sulfuric acid solution (40 to 60 °C) or a dilute alkali solution (such as an aqueous sodium hydroxide solution) to remove smuts adhered to the surface thereof. If the smuts are removed with an alkali, the aluminum alloy plates are subsequently dipped in an acid solution (such as an aqueous sulfuric acid or hydrochloric acid solution) to wash and neutralize the alloy plates.

After desmutting the surface, the aluminum alloy plates are anodized. The anodization may be carried out in a conventionally well known manner, but most useful electrolyte is sulfuric acid. Secondary preferred electrolyte is phosphoric acid. Moreover, the method using a mixed acid of sulfuric acid and phosphoric acid as an electrolyte as disclosed in J.P. KOKAI No. 55-28400(U.S. Pat. 4,229,226) is also a useful means.

In the sulfuric acid method, the treatment is generally performed using direct current, but alternating current may also be used. Sulfuric acid is used in a concentration ranging from 5 to 30% by weight and the aluminum alloy plates are electrolyzed at 20 to 60 °C for 5 to 250 seconds so as to form an anodized layer on the alloy plates in an amount ranging from 1 to 10 g/m$^2$. The electrolyte preferably comprises aluminum ions. Moreover, the current density during the anodization preferably ranges from 1 to 20 A/dm$^2$.

In the phosphoric acid method, the concentration of phosphoric acid is 5 to 50% by weight and the aluminum alloy plates are electrolyzed at 30 to 60 °C for 10 to 30 seconds at a current density of 1 to 15 A/dm$^2$. The aluminum substrate thus treated is preferably subjected to a surface-treatment with a silicate as disclosed in U.S. Patent No. 2,714,066.

The aluminum substrate may have a coating layer (hereinafter referred to as "back coat") of an organic polymer on the back face thereof for inhibiting the dissolution of the anodized layer during development and for preventing any damage of the light-sensitive layer upon putting the PS plates in piles. Materials for the back coats used are organic polymers insoluble in alkali developers such as those described in European Patent Publication No. 490,515A. The thickness of the back coat is not restricted to a specific range so far as it is sufficient for inhibiting the dissolution of the anodized layer of the aluminum during development, but preferably ranges from 0.01 to 50 $\mu$m, more preferably 0.05 to 10 $\mu$m. Any method can be adopted to apply a back coat to the back face of the aluminum substrate, but most preferred method comprises preparing a coating solution of an organic polymer, applying the solution and then drying from the viewpoint of ensuring the foregoing thickness of the back coat.

It is also preferred to apply an underlying coating of a compound listed below to the surface of the substrate. Examples of compounds for the underlying coating preferably include carboxy methyl cellulose, dextrine, gum arabic, phosphonic acids having amino groups such as 2-aminoethylphosphonic acid, organic phosphonic acids optionally having substituents such as phenylphosphonic acid, naphthylphosphonic acid, alkylphosphonic acid, glycerophosphonic acid, methylenediphosphonic acid and ethylenediphosphonic acid, organic phosphoric acids optionally having substituents such as phenylphosphoric acid, naphthylphosphoric acid, alkylphosphoric acid and glycerophosphoric acid, organic phosphinic acids optionally having substituents such as phenylphosphinic acid, naphthylphosphinic acid, alkylphosphinic acid and glycerophosphinic acid, amino acids such as glycine and $\beta$ -alanine, amine hydrochlorides having hydroxyl groups such as triethanolamine hydrochloride, water-soluble polymers having sulfonate residues as disclosed in U.S.Patent No. 4,578,342 and acidic dyes as disclosed in J.P. KOKAI No. Sho 60-64352.

The underlying coating can be formed by dissolving the foregoing compound in a solvent such as water, methanol, ethanol, methyl ethyl ketone or a mixture thereof, applied onto a substrate and then dried. A yellow dye may be incorporated into the underlying coating for improvement of the tone reproduction of the resulting PS plate.

The coated amount of the underlying coating (weighed after drying) suitably ranges from 2 to 200 $mg/m^2$, preferably 5 to 100 $mg/m^2$.

The PS plate preferably has a mat layer comprising individual projections on the light-sensitive layer.

The mat layer is applied for improving the adhesion of a negative film to the PS plate during contact exposure to light under vacuum. Thus, the time required for evacuation is reduced and breakage of fine half tone dots observed during imagewise exposure due to insufficient adhesion can be prevented.

The mat layer may be formed by any method such as a method which comprises heat-bonding spreaded solid powder to the surface as disclosed in U.S.Patent No. 5,028,512; and a method which comprises spraying a polymer-containing water and then drying as disclosed in U.S.Patent No. 4,557,994. Moreover, the mat layer per se is preferably those capable of being dissolved in an aqueous alkaline developer or capable of being removed by the developer.

The light-sensitive composition applied to a substrate is imagewise exposed to light through an original transparency carrying images such as line drawings or half tone dot images and then developed with an aqueous alkaline developer to give a relief image negative to the original.

The PS plate of the present invention can be developed with any known developer, but the following developers are preferably used.

For instance, the developer comprises, as essential components, at least one alkaline agent and water.

Examples of alkaline agents include such an inorganic alkaline agent as sodium silicate, potassium silicate, potassium hydroxide, sodium hydroxide, lithium hydroxide, sodium, potassium or ammonium tertiary or secondary phosphate, sodium metasilicate, sodium carbonate, sodium bicarbonate, potassium carbonate or ammonia; and such an organic amine compound as mono-, di- or trimethylamine, mono-, di- or triethylamine, mono- or diisopropylamine, n-butylamine, mono-, di- or triethanolamine, mono -, di- or triisopropanolamine, ethyleneimine or ethylenediamine. These alkaline agents may be used alone or in combination.

The amount of these alkaline agents used in the developer ranges from 0.05 to 10% by weight and preferably 0.5 to 5% by weight based on the total weight of the developer. If the amount is less than 0.05% by weight, the imagewise exposed PS plate is insufficiently developed, while the use of these alkaline agents in an amount higher than 10% by weight adversely affects the printing properties of the resulting lithographic printing plate.

The developer used in the invention may further comprise at least one surfactants selected from anionic surfactants and amphoteric sufactants as disclosed in J.P. KOKAI No. Sho 50-51324 and nonionic surfactants as disclosed in J.P. KOKAI Nos. Sho 59-75255 and Sho 60-111246; or a polyelectrolyte as disclosed in J.P. KOKAI Nos. 55-95946 and Sho 56-142528. Thus, the wettability and gradation density of the light-sensitive layer can be improved. Among these surfactants, preferred are anionic ones.

The amount of these surfactants and polyelectrolytes is not restricted to a specific range, but preferably ranges from 0. 003 to 5% by weight and, in particular 0.006 to 1% by weight.

The developer used for developing the PS plate of the present invention may optionally comprise a water-soluble sulfite which is preferably an alkali or alkaline earth metal salt of sulfurous acid such as sodium sulfite, potassium sulfite, lithium sulfite and magnesium sulfite. The content of these sulfites in the developer ranges from 0 to 4% by weight and preferably 0.1 to 1% by weight on the basis of the total weight of the developer.

The developer may comprise, in place of the foregoing water-soluble sulfite, alkali-soluble pyrazolone compounds, alkali-soluble thiol compounds or hydroxyl group-containing aromatic compounds such as

methyl resorcine. These compounds may be used in combination with the foregoing water-soluble sulfites.

The developer may likewise comprise other additives such as anti-foaming agents and hard water softeners. Preferred anti-foaming agents are silicone type ones and preferred hard water softeners are, for instance, polyphosphoric acid salts and aminopolycarboxylic acids. The optimum amount of the hard water softener varies depending on the hardness of water used and the amount thereof, but preferably ranges from 0.01 to 5% by weight and more preferably 0.01 to 0.5% by weight on the basis of the weight of the developer practically employed.

The developer used for the development of the PS plate of the present invention may optionally comprise specific organic solvents. Organic solvents usable are preferably those capable of dissolving or swelling the non-exposed portion (non-image area) of the foregoing light-sensitive layer if it is added to the developer and having a solubility in water of not more than 10% by weight at ordinary temperature. Specific examples of such organic solvents include carboxylic acid esters such as ethyl acetate, butyl acetate, benzyl acetate, ethylene glycol monobutyl acetate, butyl lactate and butyl levulinate; ketones such as methyl isobutyl ketone and cyclohexanone; alcohols such as ethylene glycol monobutyl ether, ethylene glycol benzyl ether, ethylene glycol monophenyl ether, benzyl alcohol, methyl phenyl carbinol, n-amyl alcohol and methylamyl alcohol; alkyl-substituted aromatic hydrocarbons and halogenated hydrocarbons. These organic solvents may be used in combination. Particularly preferred organic solvents are ethylene glycol monophenyl ether and benzyl alcohol. Preferred results are obtained when these organic solvents are added to the developer in an amount ranging from 0 to 20% by weight, in particular 2 to 10% by weight.

The developer may comprise a certain solubilizing agent to promote the dissolution of the organic solvent in water. Examples of such solubilizing agents are low molecular weight alcohols and ketones having solubilities greater than that of the organic solvent used. Alternatively, anionic and amphoteric surfactants may be used. Examples of preferred surfactants are sodium isopropylnaphthalenesulfonate, sodium N-methyl-N-pentadecylaminoacetate and sodium laurylsulfate.

However, most preferably the developer is substantially free of organic solvents since the use of organic solvents or the like accompanies various disadvantages such as problems of toxicity during working, hygienic problems (e.g., giving out of a bad smell), problems concerning safety (e.g., a fire and gas explosion), problems concerning workability (e.g., foaming), problems of environmental pollution due to waste and problems of cost.

The term "substantially free of organic solvents" herein used means that the developer does not include organic solvents to such an extent that the foregoing problems concerning environmental hygiene, safety, workability or the like arise. In the present invention, the term means that the organic solvents are included in the developer composition in an amount of not more than 2% by weight, preferably not more than 1% by weight.

Examples of such aqueous alkaline developers substantially free of organic solvents are those disclosed in U.S.Patent Nos. 4,500,625 and 4,467,027 and J.P.KOKAI No.Sho 62-24263. It is also possible to use developer compositions for developing imagewise exposed positive-working PS plates.

The PS plate of the present invention can likewise be subjected to plate-making processes such as those disclosed in U. S.Patent No. 4,291,117 and J.P. KOKAI Nos. Sho 54-8002 and Sho 59-58431. More specifically, after the developing treatment, the plate may be washed with water and then desensitized; may be desensitized without water-washing; may be treated with an aqueous solution containing an acid; or may be treated with an aqueous solution containing an acid and then desensitized.

Moreover, in the development processing of the PS plate of this kind, the alkali concentration of the developer is reduced in proportion to the amount of the PS plates processed or due to absorption of carbon dioxide present in the air when an automatic developing machine is operated over a long time period. In this case, the processing ability thereof can be recovered by the supplementation of a replenisher as disclosed in U.S.Patent No. 4,259,434. The supplementation of the replenisher is preferably carried out by the mehod disclosed in U.S. Patent No. 4,882,246.

The foregoing plate-making process is preferably performed using an automatic developing machine as disclosed in U.S.Patent No. 4,952,958 and J.P. KOKAI No. Hei 2-32357.

The desensitizing gums optionally applied to the plate at the final step of the plate-making process are preferably those disclosed in J.P. KOKOKU Nos. Sho 62-16834, Sho 62-25118 and Sho 63-52600 and J.P. KOKAI Nos. Sho 62-7595, Sho 62-11693 and Sho 62-83194.

After development processing, unnecessary portions of the image area can optionally be removed with an erasing liquid for negative or scrubbed away with a stone bar.

The lithographic printing plate obtained from the PS plate of the present invention has excellent printing durability since the diazonium compound used is a high molecular weight compound and the PS plate has good developability since the diazonium compound has alkali-soluble groups and a binder used is a highly

alkali-soluble lipophilic polymeric compound having an acid value of not less than 20 and less than 250. Thus the printing plate hardly causes background contamination during printing. In particular, the PS plate is developable with an alkaline developer free of organic solvents.

The present invention will hereinafter be explained in more detail with reference to the following non-limitative working Examples and the effects practically accomplished by the invention will also be discussed in comparison with Comparative Examples.

Example 1

A rolled plate of a JISA1050 aluminum material which comprised 99.5% Al, 0.01% Cu, 0.03% Ti, 0.3% Fe and 0.1% Si and had a thickness of 0.24 mm was subjected to surface-graining using a rotary nylon brush (6-10 nylon) and a 20% by weight aqueous suspension of pumice stone (available from Kyoritsu Ceramic Materials Co., Ltd.) and then sufficiently washed with water.

The aluminum plate was etched by immersing in a 15% by weight aqueous solution of sodium hydroxide (containing 5% by weight of Al) till 5 g/m$^2$ of Al was dissolved and then washed with running water. After neutralizing with a 1% by weight nitric acid solution, the plate was electrolytically surface-roughened in a 0.7% by weight aqueous nitric acid solution (containing 0.5% by weight of Al) using an alternating voltage having a rectangular waveform at 10.5 V of anodic voltage and 9. 3 V of cathodic voltage such that the quantity of electricity at the anode time was 160 coulomb/dm$^2$ (current ratio: r = 0.90; the current waveform described in Examples of J.P. KOKOKU No. Sho 58-5796). After water-washing, the plate was etched by immersing in a 10% by weight aqueous solution of sodium hydroxide till 1 g/m$^2$ of Al was dissolved and then washed with water. Then the plate was immersed in a 30% aqueous sulfuric acid solution maintained at 50 °C to perform desmutting and washed with water.

The aluminum plate was then treated in a 20% by weight aqueous solution of sulfuric acid (containing 0.8% by weight of Al) maintained at 35 °C using a DC to form a porous anodized layer. More specifically, the plate was electrolyzed at a current density of 13 A/dm$^2$ and the electrolyzation time was adjusted so that the weight of the anodized layer reached 2.0 g/m$^2$. After water-washing, the plate was immersed in a 3% aqueous solution of sodium silicate at 70 °C for 30 seconds, washed with water and then dried.

The aluminum substrate thus obtained had a reflection density of 0.28 as determined by a Macbeth RD 920 reflection densitometer and a central line-averaged surface roughness of 0. 54 $\mu$m.

Then an underlying coating consisting of 2-acrylamide-2-methylpropane sulfonic acid copolymer was applied to the substrate in the method described in Example 1 of U.S.Patent No. 4,578,341.

Further the following light-sensitive solution-1 was applied to the surface of the substrate using a bar coater and dried at 120 °C for 30 seconds. The coated amount was 1.7 g/m$^2$ (weighed after drying).

| (Light-Sensitive Solution-1) | |
|---|---|
| diazonium compound-1 | 1.2 g |
| lipophilic polymeric compound-1 | 5.0 g |
| oil-soluble dye (Victoria Pure Blue BOH) | 0.15 g |
| fluorine atom-containing surfactant (Megafac F-177, available from Dainippon Ink and Chemicals, Inc.) | 0.02 g |
| tricresyl phosphate | 0.2 g |
| phosphorous acid | 0.03 g |
| malic acid | 0.03 g |
| half ester of styrene/maleic anhydride copolymer with n-hexyl alcohol | 0.05 g |
| 2-methoxypropanol | 50 g |
| methanol | 20 g |
| methyl ethyl ketone | 20 g |
| methyl lactate | 10 g |

After the application of the light-sensitive layer, an aqueous solution containing 20% by weight of copolymer consisting of methyl methacrylate, ethylacrylate and 2-acrylamide-2-methylpropanesulfonic acid sodium salt with a molar ratio of 50:30:20 was applied to the surface thereof through the electrostatic spray and dried for 5 seconds in an atmosphere of 60 °C to perform matting of the surface.

The foregoing processes for treating the substrate and coating processes were performed according to a continuous-through production line. In addition, the PS plate was covered with an interleaf for protecting

the surface thereof. The surface of the interleaf used had been processed with polyethylene.

The negative-working PS plate thus prepared was imagewise exposed to light for one minute at a distance of 1 m using PS Light available from Fuji Photo Film Co., Ltd.

Then the imagewise exposed PS plate was developed under the following conditions. First, the following developer-1, water and Finisher FP-2 (available from Fuji Photo Film Co., Ltd. ) were respectively charged in a first bath, a second bath and a third bath of an automatic developing machine STABLON 900 D (available from Fuji Photo Film Co., Ltd.) and the temperature of the developer and developing time were set at 30 °C and 12 seconds respectively. Then the foregoing PS plate and an imagewise exposed PS plate (a positive-working PS plate FPS-3, available from Fuji Photo Film Co., Ltd.) were cut into sheets having a size of medium octavo and alternatively developed over 6 hours in proper time intervals (30 sheets each).

| (Developer-1) | |
|---|---|
| 1K potassium silicate | 60 g |
| potassium hydroxide | 12 g |
| boron atom-containing surfactant (Emulbon T-20, available from Toho Chemical Industry Co., Ltd.) | 0.1 g |
| chelating agent (EDTA) | 0.1 g |
| silicon atom-containing anti-foaming agent (TSA-731, available from Toshiba Silicone Co., Ltd.) | 0.01 g |
| cobalt acetate | 0.04 g |
| water | 1200 g |

The pH value of the developer-1 was 13.1. When the developer was deteriorated due to the processing of these PS plates or the lapse of time and the developing ability of the developer-1 was reduced, the developing ability thereof was recovered by supplementation of the following replenisher-1.

| (Replenisher-1) | |
|---|---|
| 1K potassium silicate | 60 g |
| potassium hydroxide | 42 g |
| boron atom-containing surfactant (Emulbon GB-24F, available from Toho Chemical Industry Co., Ltd.) | 0.03 g |
| chelating agent (Feriox 115, available from Lion Corporation) | 3.5 g |
| water | 1200 g |

It was found that the developer maintained approximately the same level of developing ability during these treatments. Each PS plate was printed with Step Tablet (optical density of initial step 0.10; density difference between neighbouring two steps 0.15; step number 15). The fluctuation in solid step numbers was within one step.

The lithographic printing plate obtained by imagewise exposing and developing the foregoing negative-working PS plate was fitted to a printing press SOR-M (available from Heidelberg Company) and printing operation was performed using Graph G (available from Dainippon Ink and Chemicals, Inc.) as an ink, a mixture of water, isopropanol (10%) and EU-3 (1%; available from Fuji Photo Film Co., Ltd.) as a dampening water and coated paper. The results obtained are summarized in the following Table 2.

Example 2

To the back face of an aluminum substrate which had been subjected to surface treatments identical to those performed in Example 1, there was applied a back coat solution of 0.2 part by weight of a polyurethane resin (Estane #5715, available from Monsanto Chemical Company) in a mixture of 16 parts by weight of ethylene glycol monomethyl ether and 24 parts by weight of methyl ethyl ketone in an amount of 0.2 $g/m^2$ (weighed after drying), followed by drying. Further an underlying coating identical to that used in Example 1 was applied onto the upper face of the substrate.

Further the following light-sensitive solution-2 was applied to the surface of the substrate using a bar coater and dried at 120 °C for 25 seconds. The coated amount was 1.6 $g/m^2$ (weighed after drying).

| (Light-Sensitive Solution-2) | |
|---|---|
| diazonium compound-2 | 1.2 g |
| lipophilic polymeric compound-1 | 5.0 g |
| Victoria Pure Blue BOH | 0.15 g |
| Megafac F-177 | 0.02 g |
| tricresyl phosphate | 0.2 g |
| phenylphosphonic acid | 0.03 g |
| dipicolinic acid | 0.03 g |
| phosphorous acid | 0.03 g |
| pivalate of p-hydroxystyrene (degree of esterification: 50%) | 0.1 g |
| 2-methoxypropanol | 50 g |
| methanol | 18 g |
| methyl ethyl ketone | 20 g |
| methyl lactate | 10 g |
| water | 2 g |

After the application of the light-sensitive layer, the surface of the light-sensitive layer was matted in the same manner used in Example 1.

The negative-working PS plate thus prepared was imagewise exposed to light in the same manner used in Example 1 and then developed under the following conditions. First, the developer-1 used in Example 1, water and a rinsing solution FR-3 (available from Fuji Photo Film Co., Ltd.) were respectively charged in a first bath, a second bath and a third bath of an automatic developing machine STABLON 900 D and the foregoing PS plate and an imagewise exposed PS plate (a positive-working PS plate FPS-3) were developed in the same manner used in Example 1. When the developing ability of the developer was reduced, the following replenisher-2 was supplemented.

| (Replenisher-2) | |
|---|---|
| 1K potassium silicate | 120 g |
| potassium hydroxide | 25 g |
| chelating agent (NTA-C, available from Chelest Chemical Company) | 0.3 g |
| water | 1200 g |

When the lithographic printing plate thus obtained had unnecessary portions or film edge portions, they were removed with a deletion liquid RN-2 (available from Fuji Photo Film Co., Ltd.) for negative-working PS plates and with an erasing liquid RP-2 for positive-working PS plates. After washing away the erasing liquid, the printing plate was desensitized with Gum GU-7 (available from Fuji Photo Film Co., Ltd.) using Gum Coater G 800 (available from Fuji Photo Film Co., Ltd.). Printing operations were carried out in the same manner used in Example 1 using the resulting lithographic printing plate. The results obtained are summarized in the following Table 2.

Example 3

The same procedures used in Example 1 were repeated except that the following light-sensitive solution-3 was substituted for the light-sensitive solution-1 used in Example 1 to give a PS plate.

| (Light-Sensitive Solution-3) | |
|---|---|
| diazonium compound-3 | 1.2 g |
| 4-methoxy-2-hydroxybenzophenone-5-sulfonate of condensate of p-diazodiphenylamine with formaldehyde | 0.05 g |
| lipophilic polymeric compound-1 | 5.0 g |
| Victoria Pure Blue BOH | 0.15 g |
| Megafac F-177 | 0.02 g |
| tricresyl phosphate | 0.2 g |
| malic acid | 0.03 g |
| anionic surfactant (Pelex NBL, available from Kao Atlas Co., Ltd.) | 0.1 g |
| polyacrylic acid (Julimer AC-10L) | 0.3 g |
| half ester of styrene/maleic anhydride copolymer with n-hexyl alcohol | 0.05 g |
| 2-methoxypropanol | 50 g |
| methanol | 20 g |
| methyl ethyl ketone | 20 g |
| methyl lactate | 10 g |

After imagewise exposure to light in the same manner used in Example 1, the exposed PS plate was developed under the following conditions. First, the developer-1 used in Example 1 and Finisher disclosed below were respectively charged in a first bath and a second bath of an automatic developing machine 800E II B (available from Fuji Photo Film Co., Ltd.) and the temperature of the developer and developing time were set at 25 °C and 30 seconds respectively. Then the foregoing PS plate and an imagewise exposed PS plate (FPS-3) were cut into sheets having a size of medium octavo and alternatively developed over 3 hours in proper time intervals (30 sheets each). When the developing ability of the developer was reduced, the foregoing replenisher-1 was supplemented. The supplementation was performed in such a manner that 390 ml of the replenisher was added every one hour and that 37 ml of the replenisher was added each time a PS plate was processed.

Thus, almost the same results were obtained and it was confirmed that the developer maintained approximately the same level of developing ability throughout the development processing.

Finisher

| Component | Amount (part by weight) |
|---|---|
| Pure Water | 750 |
| Gum arabic | 20 |
| Enzyme modified potato starch (viscosity (20%): 10 cps at 20 °C) | 200 |
| Enzyme modifid corn starch (viscosity (20%): 5 cps at 20 °C) | 40 |
| Phosphated waxy starch (viscosity (20%): 40 cps at 20 °C) (phosphorus bonded per unit: 1.0) | 20 |
| Sodium dilauryl sulfosuccinate | 10 |
| Ammonium phosphate | 3 |
| Phosphoric acid (85%) | 4 |
| EDTA-4Na | 3 |
| Ethylene glycol | 20 |
| Benzyl alcohol | 25 |
| Sodium dehydroacetate | 0.5 |
| Emulsion type silicon atom-containing antifoaming agent | 0.2 |

After the starches were dissolved in hot water at 80 ° C and cooled, the other components were added and dissolved with stirring to prepare a plate finisher.

Printing operations were carried out in the same manner used in Example 1 using the resulting lithographic printing plate. The results obtained are summarized in the following Table 2.

Examples 4 to 13

The same procedures used in Example 1 were repeated except that, in the light-sensitive solution-1, the diazonium compounds 4 to 13 were substituted for the diazonium compound 1 used in Example 1 to give PS plates. Then printing operations were carried out in the same manner used in Example 1 using the lithographic printing plates obtained by imagewise exposing and developing these PS plates in the same manner used in Example 1. The results obtained are summarized in the following Table 2.

Examples 14 to 17

The same procedures used in Example 1 were repeated except that, in the light-sensitive solution-1, the lipophilic polymeric compounds 2 to 5 were substituted for the lipophilic polymeric compound 1 used in Example 1 to give PS plates. Then printing operations were carried out in the same manner used in Example 1 using the lithographic printing plates obtained by imagewise exposing and developing these PS plates in the same manner used in Example 1. The results obtained are summarized in the following Table 2.

Example 18

The same procedures used in Example 1 wre repeated except that the following developer-2 and replenisher-3 were respectively substituted for the developer-1 and replenisher-1 used in Example 1 to give a PS plate. Printing operations were carried out in the same manner used in Example 1 using the lithographic printing plate obtained by imagewise exposing and developing the PS plate in the same manner used in Example 1. The results obtained are summarized in the following Table 2.

| (Developer-2) | | |
|---|---|---|
| sodium carbonate· monohydrate | | 5.9 g |
| sodium bicarbonate | | 3.2 g |
| anionic surfactants | Pelex NBL<br>Newcoal B4SN | 15.3 g<br>2.7 g |
| EDTA<br>water | | 0.8 g<br>300 g |
| (pH: 9.9) | | |

| (Replenisher-3) | | |
|---|---|---|
| sodium carbonate· monohydrate | | 7.4 g |
| sodium bicarbonate | | 1.0 g |
| anionic surfactants | Pelex NBL<br>Newcoal B4SN | 25.0 g<br>2.7 g |
| EDTA<br>water | | 0.8 g<br>250 g |
| (pH: 10.85) | | |

Example 19

A JISA 1050 aluminum plate having a thickness of 0.3 mm was etched at 65 °C for one minute in a 5% by weight aqueous solution of sodium hydroxide, washed with water, neutralized by immersing in a 10% by weight aqueous nitric acid solution at 25 °C for one minute and then washed with water.

The aluminum plate was electrolytically surface-roughened at a current density of 50 A/dm$^2$ using an AC for 30 seconds in a 1% by weight aqueous nitric acid solution maintained at 30 °C, washed with water for 10 seconds in a washing bath and then immersed in a 10% by weight aqueous sodium hydroxide solution to etch the plate so that the amount of dissolved aluminum reached 1 g/m$^2$.

Then the plate was anodized at 25 °C and 6 A/dm$^2$ in a 20% aqueous sulfuric acid solution while the electrolyzing time was controlled in such a manner that the weight of the resulting oxidized layer was 1.2 g/m$^2$. The plate was exposed to water vapor (100% humidity, temperature 98 °C) for 20 seconds to perform a sealing treatment.

The plate was then immersed in a 2.5% aqueous sodium silicate solution maintained at 70 °C for one minute, washed with water and dried. The aluminum substrate thus obtained had a reflection density of 0.23 and a central line-averaged surface roughness of 0.42 µm.

A PS plate was prepared by applying an underlying coating and a light-sensitive layer onto the substrate and then matting the plate in the same manner used in Example 1. Then the resulting PS plate was imagewise exposed to light as in Example 1.

Then a horizontally conveying and developer-throw-away type automatic developing machine as disclosed in U.S.Patent No. 4,737,810 was provided and the developer used in Example 1 and 8ℓ of a rinsing solution having the following formulation and diluted 8 times with water were introduced into a first bath and a second bath of the machine respectively.

Formulation of Rinsing Solution

| | |
|---|---|
| phosphoric acid (85% by weight aqueous solution) | 48 g |
| sodium hydroxide | 19.2 g |
| sodium dioctylsulfosuccinate | 24 g |
| sodium dodecylphenyl ether disulfonate | 24 g |
| silicon atom-containing anti-foaming agent TSA-731 | 0.08 g |
| water | 875 g |

The foregoing imagewise exposed PS plate was developed in this automatic developing machine. Unnecessary portions were removed using a deletion liquid RN-2 (available from Fuji Photo Film Co., Ltd.). Any residual film was not observed in the erased portions. After water-washing, the resulting plate was desensitized with Gum GU-7.

Printing operations were carried out in the same manner used in Example 1 using the lithographic printing plate thus obtained. The results obtained are summarized in Table 2.

Comparative Examples 1 and 2

The same procedures used in Example 1 were repeated except that, in the light-sensitive solution used in Example 1, a diazonium compound 14 or 15 was substituted for the diazonium compound-1 to give PS plates. Printing operations were carried out in the same manner used in Example 1 using the lithographic printing plates obtained by imagewise exposing and developing the PS plate in the same manner used in Example 1. The results obtained are summarized in the following Table 2.

Comparative Examples 3 and 4

The same procedures used in Example 1 were repeated except that, in the light-sensitive solution used in Example 1, a lipophilic polymeric compound 6 or 7 was substituted for the lipophilic polymeric compound-1 to give PS plates. Printing operations were carried out in the same manner used in Example 1 using the lithographic printing plates obtained by imagewise exposing and developing the PS plate in the same manner used in Example 1. The results obtained are summarized in the following Table 2.

Table 2

| Ex. No. | Diazonium Compound Used | Lipophilic Compound Used | Developer Used | Results of Printing Contamination | Printing Durability (No.) |
|---|---|---|---|---|---|
| 1 | 1 | 1 | 1 | A | 65,000 |
| 2 | 2 | 1 | 1 | A | 55,000 |
| 3 | 3 | 1 | 1 | B | 65,000 |
| 4 | 4 | 1 | 1 | A | 60,000 |
| 5 | 5 | 1 | 1 | B | 70,000 |
| 6 | 6 | 1 | 1 | A | 60,000 |
| 7 | 7 | 1 | 1 | A | 55,000 |
| 8 | 8 | 1 | 1 | B | 75,000 |
| 9 | 9 | 1 | 1 | B | 55,000 |
| 10 | 10 | 1 | 1 | B | 55,000 |
| 11 | 11 | 1 | 1 | A | 55,000 |
| 12 | 12 | 1 | 1 | A | 60,000 |
| 13 | 13 | 1 | 1 | A | 60,000 |
| 14 | 1 | 2 | 1 | A | 65,000 |
| 15 | 1 | 3 | 1 | A | 55,000 |
| 16 | 1 | 4 | 1 | B | 60,000 |
| 17 | 1 | 5 | 1 | B | 65,000 |
| 18 | 5 | 1 | 2 | B | 70,000 |
| 19 | 1 | 1 | 1 | B | 60,000 |
| 1* | 14 | 1 | 1 | D | 35,000 |
| 2* | 15 | 1 | 1 | A | 20,000 |
| 3* | 1 | 6 | 1 | D | 55,000 |

*: Comparative Example

Degree of Contamination:

A: no contamination B: almost no contamination

C: slightly contaminated D: severely contaminated

As seen from the results listed in Table 2, the lithographic printing plates prepared from the PS plates of the present invention (Examples 1 to 19) do not exhibit background contamination during printing and have excellent printing durability as compared with those of Comparative Examples and, therefore, the PS plate of the present invention has high commercial value.

## Claims

**1.** A presensitized plate for use in making a lithographic printing plate comprising a substrate provided thereon with a light-sensitive layer formed from a light-sensitive composition comprising a diazonium polymeric compound having repeating units represented by the following general formulas (I) and (II) and a lipophilic polymeric compound having an acid value of not less than 20 and less than 250:

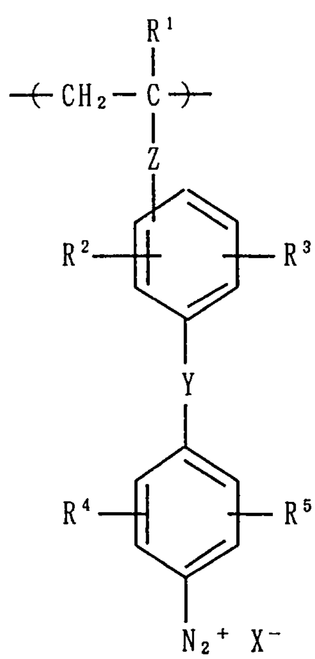

(I)

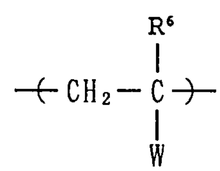

(II)

wherein $R^1$ and $R^6$ each represents a hydrogen atom or a methyl group; Z represents a divalent linking group; $R^2$, $R^3$, $R^4$ and $R^6$ may be the same or different and each represents a hydrogen or halogen atom or an alkyl, alkoxy or hydroxyl group; Y represents -NH-, -O- or -S-; $X^-$ represents an anion; W represents a carboxyl group, a group having at least one carboxyl group, a group having at least one sulfonate residue or a group having at least one phosphorus atom-containing oxyacid residue.

**2.** The presensitized plate of claim 1 wherein the phosphorus atom-containing oxyacid residue is selected from the group consisting of those represented by the following general formulas (III) and (IV):

$-O-PO(OR^7)(OR^8)$ (III)

-PO($OR^7$)($OR^8$)   (IV)

wherein $R^7$ is a hydrogen atom; and $R^8$ represents a hydrogen atom or an alkyl or aryl group.

3. The presensitized plate of claim 1 wherein, in the foregoing repeating units represented by Formula (I), Z is a divalent linking group which forms the polymerizable moiety having an acrylic acid, methacrylic acid or styrene skeleton;$R^2$, $R^3$, $R^4$ and $R^5$ may be the same or different and each represents a hydrogen, chlorine or bromine atom, an alkyl or alkoxy group having 1 to 8 carbon atoms or a hydroxyl group; Y is -NH-; $X^-$ is an anion selected from the group consisting of those derived from hexafluorophosphoric acid, dibutylnaphthalenesulfonic acid, dodecylbenzenesulfonic acid and 2-methoxy-4-hydroxy-5-benzoylbenzenesulfonic acid.

4. The presensitized plate of claim 1 wherein the copolymerization ratio of the repeating unit (I) to the repeating unit (II) ranges from 1:0.2 to 0.2:1 expressed in terms of charge ratio.

5. The presensitized plate of claim 1 wherein the acid content of the diazonium compound ranges from 0.5 to 5 meq/g expressed in terms of the amount of the alkali-soluble groups included in 1g of the compound.

6. The presensitized plate of claim 1 wherein the weight-average molecular weight of the diazonium compound ranges from about 1,500 to 50,000.

7. The presensitized plate of claim 1 wherein the light-sensitive composition comprises 1 to 70% by weight of the diazonium compound on the basis of the weight of the solid contents of the composition.

8. The presensitized plate of claim 1 wherein the lipophilic polymeric compound is selected from the group consisting of copolymers having structural units derived from monomers selected from those listed below and having an acid value of not less than 20 and less than 250:

(meth)acrylamides, (meth)acrylic acid esters and hydroxystyrenes having aromatic hydroxy groups; unsaturated sulfonamides; (meth)acrylic acid esters having an aliphatic hydroxy groups; unsaturated carboxylic acids; (substituted) acrylates; (substituted) methacrylates; (meth)acrylamides; vinyl ethers; vinyl esters; styrenes; vinyl ketones; olefins; N-vinyl pyrrolidone, N-vinyl carbazole, 4-vinyl pyridine, acrylonitrile and methacrylonitrile; and unsaturated imides.

9. The presensitized plate of claim 8 wherein the lipophilic polymeric compound has a molecular weight ranging from 10,000 to 150,000.

10. The presensitized plate of claim 1 wherein the lipophilic polymeric compound is incorporated into the light-sensitive composition in an amount ranging from 40 to 99% by weight on the basis of the weight of the solid contents of the composition.

European Patent Office

# EUROPEAN SEARCH REPORT

Application Number

EP 92 11 5108

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y<br><br>D | DE-A-3 343 536 (FUJI PHOTO FILM CO LTD)<br>* the whole document *<br>& US-A-4 581 313<br>--- | 1-10 | G03F7/021 |
| Y | EP-A-0 415 422 (FUJI PHOTO FILM CO LTD)<br>* page 7, line 38 - page 8, line 30 *<br>--- | 1-10 | |
| Y | PATENT ABSTRACTS OF JAPAN<br>vol. 12, no. 380 (P-769)(3227) 12 October 1988<br>& JP-A-63 127 235 ( FUJI PHOTO FILM CO LTD ) 31 May 1988<br>* abstract *<br>--- | 1-10 | |
| Y | PATENT ABSTRACTS OF JAPAN<br>vol. 12, no. 422 (P-783)(3269) 9 November 1988<br>& JP-A-63 157 144 ( DAINIPPON INK & CHEM INC ) 30 June 1988<br>* abstract *<br>--- | 1-10 | **TECHNICAL FIELDS SEARCHED (Int. Cl.5 )** |
| A | PATENT ABSTRACTS OF JAPAN<br>vol. 7, no. 241 (P-232)(1386) 2 October 1983<br>& JP-A-58 127 923 ( FUJI SHASHIN FILM K.K. ) 30 July 1983<br>* abstract *<br>----- | 1 | G03F |

The present search report has been drawn up for all claims

| Place of search | Date of completion of the search | Examiner |
|---|---|---|
| THE HAGUE | 16 OCTOBER 1992 | Martine LUDI |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)